# EUROPEAN PATENT SPECIFICATION

(11) **EP 0 448 998 B1**
(45) Date of publication and mention of the grant of the patent: **24.05.1995**
(21) Application number: 91103404.9
(22) Date of filing: 06.03.1991
(51) Int. Cl.: C07C 43/04, C07C 41/06

(54) **Production of ethyl tertiary alkyl ethers**
Herstellung von Ethyl-tert.alkyläthern
Préparation d'éthyl terioalkyl éthers

(30) Priority: 28.03.1990 US 500356
(43) Date of publication of application: 02.10.1991
(73) Proprietor: MOBIL OIL CORPORATION, Fairfax, Virginia 22037-0001 (US)
(72) Inventor: Harandi, Mohsen Madimi, Lawrenceville, NJ 08648 (US); Owen, Hartley, Belle Meade, NJ 08502 (US)
(74) Representative: Kador & Partner

(56) References cited:
- EP-A- 0 289 232
- EP-A- 0 323 138
- EP-A- 0 366 501
- WO-A-89/09760
- US-A- 4 316 724
- US-A- 4 413 150
- US-A- 4 827 046

## Description

This invention relates to production of lower ethyl isoalkyl ethers, in particular to an integrated system for converting crude ethanol to valuable products by etherification with lower branched olefins such as C₄-C₅ isoolefins.

Increasing demand for high octane gasolines blended with lower aliphatic alkyl ethers as octane boosters and supplementary fuels has created a significant demand for high octane tertiary alkyl ethers, including the C₆ and C₇ ethyl alkyl ethers such as ethyl tertiary-butyl ether (ETBE) and ethyl t-amyl ether (ETAE). H. E. Buc et al., S.A.E. Journal (Transactions), Vol. 39, No. 3, p. 333, discuss the advantages of using such materials to enhance gasoline octane.

Ethanol may be readily obtained from biomass by fermentation in a known manner. Crude ethanol from such processes usually contains a significant amount of water, from minor amounts up to 90 wt%. Large amounts of water are not generally desirable in etherification, due mainly to hydration of olefins.

Processes for preparation of methyl-t-alkyl ethers from methanol feedstocks containing 4 to 20 wt% of water, which include extracting the methanol from its aqueous solution with a C₄+ olefin-containing extractant, followed by etherification of the extracted hydrocarbon phase, are disclosed in EP-A-289,232, US-A-4,827,046 and WP-A-89/09760.

US-A-4,316,724 relates to a method of forming single phase gasoline composition from blends of gasoline and aqueous ethanol, whereby ethanol is converted into ether and the water is converted into tertiary alcohol in the presence of etherification-alcoholification catalyst.

It is an object of the present invention to provide a novel and economic technique for removing excess water from crude ethanol feedstocks, including novel operating methods for treating the oxygenate feedstocks prior to etherification.

According to the present invention a process for producing ethyl tertiary alkyl ethers from crude aqueous ethanol comprises passing the crude ethanol to a distillation column to provide an overhead stream containing a major amount of ethanol and a minor amount of water, contacting said overhead stream with a liquid hydrocarbon extractant rich in C₄+ isoalkene under liquid extraction conditions to provide an aqueous phase containing a minor amount of ethanol and a major amount of water and an organic phase containing the extractant and the remaining ethanol and substantially free of water, recycling the aqueous phase as reflux to the distillation column and contacting ethanol and C₄+ isoalkene from said organic phase with an acid etherification catalyst under substantially anhydrous reaction conditions to produce an effluent containing ethyl tertiary alkyl ether.

The overhead stream typically contains at lease 5 wt% water, the organic phase not more than about 1.5 wt% water, based on ethanol. The effluent from the etherification may be distilled to recover ethyl t-alkyl ether in a liquid product stream, unreacted C₄ light hydrocarbon and ethanol being recovered in an overhead stream which is contacted in an effluent washer with liquid water bottoms from said distillation column to wash ethanol from the effluent, ethanol-rich wash water from the effluent washer being returned to the column for codistillation with crude aqueous ethanol. The liquid product stream containing ethyl t-alkyl ether may also contain C₅+ hydrocarbon.

The crude aqueous ethanol may contain up to 90 wt% water. The extractant advantageously comprises a major proportion of C₄-C₅ isoalkenes, and may be derived from FCC light naphtha. The crude aqueous ethanol may be derived from biomass.

A continuous feedstock separation and etherification reactor system for converting crude ethanol feedstock to ethyl t-alkyl ether comprises prefractionator means for enriching ethanol in a distillation tower; means for recovering said enriched ethanol as an overhead distillate stream; means for mixing said enriched ethanol distillate stream with a liquid hydrocarbon extraction stream; extractor means for contacting the enriched liquid containing a minor amount of water with the liquid hydrocarbon extraction stream under extraction conditions favorable to selective extraction of ethanol, thereby providing an extract liquid stream rich in ethanol and an aqueous raffinate stream lean in ethanol; means for recycling the raffinate stream as reflux to the prefractionator distillation tower; catalytic reactor means operatively connected for contacting the extract stream in a catalytic reaction zone with acid etherification catalyst under process conditions to convert ethanol to ether; product separation means for distilling etherification effluent to recover ethyl t-alkyl ether in a liquid product stream and unreacted C₄ light hydrocarbon and ethanol in an overhead stream; effluent washer means for the contacting etherification effluent overhead stream with at least a portion of liquid water bottoms from the prefractionator means to wash ethanol from the light hydrocarbon effluent; and means for passing ethanol-rich wash water from the effluent washer for codistillation with crude ethanol feedstock. Considerable advantage results from the fact that the prefractionation step is operatively connected with the extraction step to provide means for recycling the extraction raffinate stream as reflux to the prefractionator distillation tower. This technique is particularly useful in obtaining ethanol for etherification containing less than 1.5 wt% water, which cannot be achieved by conventional distillation due to the formation of an azeotrope. The present invention is useful for removing water present in small or large amounts in the feedstock.

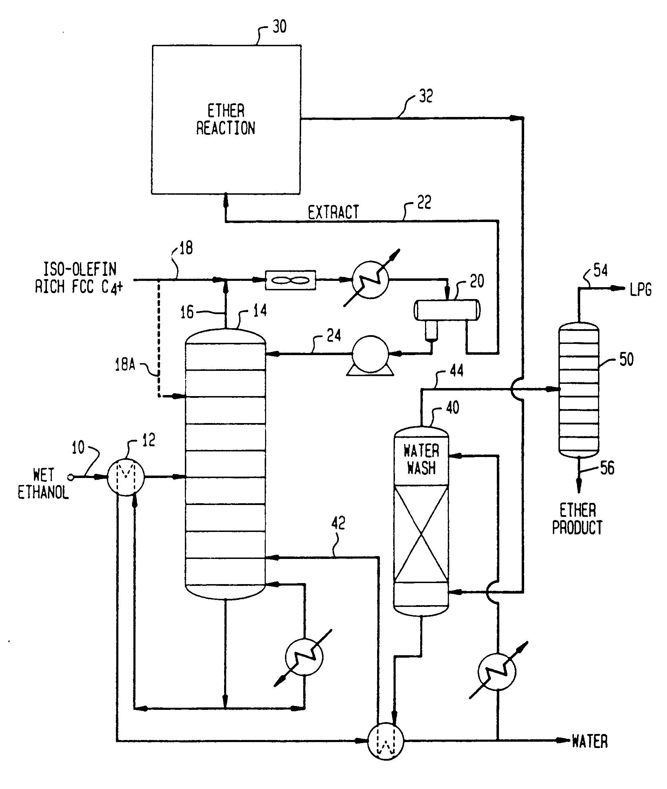

The single Figure of drawings is a schematic flowsheet depicting a preferred embodiment of the invention.

Typical feedstock materials for etherification reactions include olefinic streams, such as FCC light naphtha and butenes rich in isoolefins. These aliphatic streams are produced in petroleum refineries by catalytic cracking of gas oil or the like. The crude ethanol commercially available from fermentation processes usually contains 50 to 90 wt% water, which must be removed, preferably to an ethanol purity of about 98.5 to 99.5 wt%. It has been found that essentially the entire crude feedstock ethanol content can be recovered by liquid extraction with light olefinic liquid extractant, such as butenes and C₅+ light olefinic naphtha. The typical feed ratio range is about 9 to 20 parts hydrocarbon extractant per part by volume of ethanol.

Referring to the drawing, a continuous stream of crude wet ethanol feedstock containing 80 wt% water is introduced via conduit 10 and heat exchanger 12 to a prefractionation distillation column 14 for enriching the ethanol. The overhead vapor stream 16 from tower 14, which can be recovered as an azeotropic mixture of alcohol with at least 5 wt% water, is combined with a liquid hydrocarbon stream 18 rich in isobutylene and/or isoamylene and other C₄+ aliphatic components, preferably comprising olefinic crackate light naphtha from a fluidized catalytic cracker.

The combined stream is cooled and passed to extraction unit 20 under extraction conditions, where the liquid phases are separated into an ethanol-rich organic liquid extract stream 22 and aqueous raffinate stream 24 containing a major amount of the water present in the crude feedstream.

The aqueous raffinate stream 24 consists essentially of water, partitioned ethanol and a trace of hydrocarbon. The raffinate provides a reflux for tower 14 where it is introduced in an upper stage. The lighter organic extract phase containing hydrocarbon extraction solvent and the major amount of feedstock ethanol can be recovered from extraction unit 20, preferably with not more than 1.5 wt% water, and introduced under temperature and process conditions suitable for anhydrous conversion of ethanol in contact with etherification catalyst in reactor system 30. From the reactor system 30, the effluent product stream passes to product recovery via line 32. The etherification effluent comprises C₆+ ether, unreacted C₄+ hydrocarbons and ethanol which are passed via line 32 to a washing tower 40 for contact with water derived from prefractionation tower 14 or from fresh makeup. The washwater aqueous phase bottom stream contains residual amounts of ethanol which can be recycled via line 42, preferably to a lower stage of distillation tower 14. The washed organic liquid phase is passed via line 44 to debutanizer distillation tower 50 where ether product is removed from the system via line 56, along with any unreacted C₅+ hydrocarbon in feedstream 18.

Alternatively, it may be desirable to place the debutanizer unit 50 upstream of washing unit 40 in order to reduce the amount of hydrocarbon from the wash tower.

Unreacted C₄ hydrocarbons, such as butanes, butenes, etc., are recovered via line 54 substantially free of ethanol.

The typical preferred ethanol feedstream material is an azeotropic mixture containing about 5 to 55 volume percent water. The extraction contact unit may be a stirred multi-stage vertical extraction column adapted for continuous operation at elevated pressure. Any suitable extraction equipment may be employed, including cocurrent, cross-current or single contactors, wherein the ethanol feedstream is intimately contacted with a substantially immiscible liquid hydrocarbon solvent, which may be a mixture of C₄+ aliphatic components including lower alkanes, n-alkenes or relatively pure isoalkenes, such as isobutylene, etc. This unit operation is described in Kirk-Othmer Encyclopedia of Chemical Technology (Third Ed.), 1980, pp. 672-721. Other equipment for extraction is disclosed in US-A-4,827,046 and 4,334,890. The ethanol extraction step can be performed advantageously in a countercurrent multistage design, such as a simple packed column, rotating disk column, agitated column with baffles or mesh, or a series of single stage mixers and settlers.

In a typical ethanol extraction with FCC light naphtha, in a liquid-liquid contact and separation unit for extracting crude ethanol containing 10 wt% water at about 38^{o}C, the extractor unit and water wash unit are operated at about 35-80^{o}C (95-175^{o}F) and 100-1000 kPa. The ethanol distillation tower operates at about 100-500 kPa with overhead at about 38-58^{o}C and bottoms at about 90-150^{o}C.

The reaction of ethanol with isobutylene and isoamylenes at moderate conditions over an acid catalyst is known. The etherification catalyst employed is preferably an ion exchange resin in the hydrogen form, or an acid zeolite, but numerous acidic catalysts may be employed with varying degrees of success. Typical acidic solid catalysts include sulfonic resins, phosphoric acid modified kieselguhr, silica-alumina and medium pore acid zeolites. Typical acid catalysts include Amberlyst 15 sulfonic acid resin and various acid zeolites, such as zeolite Beta. Processes for producing and recovering ETBE and other lower alkyl t-alkyl ethers from C₄-C₇ iso-olefins are known, and disclosed for instance in US-A-4,542,250, 4,605,787, 4,814,519.

A typical etherification reaction employing the extract phase from the extraction operation described above is provided by reacting the organic stream containing isobutene and ethanol at about 75% conversion in contact with polymeric sulfonic acid resid catalyst (e.g., Amberlyst 15) at about 65^{o}C and 1100 kPa.

The present invention is particularly advantageous in its economic dewatering of crude ethanol, thus avoiding expensive and energy-intensive treatment of azeotropic mixtures produced by distillation. By extracting ethanol from the aqueous distillate with hydrocarbon reactant and recycling the raffinate, an effective technique for enriching ethanol azeotrope is achieved.

## Claims

1. A process for producing ethyl tertiary alkyl ethers from crude aqueous ethanol, which comprises passing the crude ethanol to a distillation column to provide an overhead stream containing a major amount of ethanol and a minor amount of water, contacting said overhead stream with a liquid hydrocarbon extractant rich in C₄+ isoalkene under liquid extraction conditions to provide an aqueous phase containing a minor amount of ethanol and a major amount of water and an organic phase containing the extractant and the remaining ethanol and substantially free of water, recycling the aqueous phase as reflux to the distillation column and contacting ethanol and C₄+ isoalkene from said organic phase with an acid etherification catalyst under substantially anhydrous reaction conditions to product an effluent containing ethyl tertiary alkyl ether.

2. A process according to claim 1 wherein said overhead stream contains at least 5 wt% water, and said organic phase contains not more than about 1.5 wt% water, based on ethanol.

3. A process according to any preceding claim wherein the effluent from the etherification is distilled to recover ethyl t-alkyl ether in a liquid product stream, unreacted C₄ light hydrocarbon and ethanol being recovered in an overhead stream which is contacted in an effluent washer with liquid water bottoms from said distillation column to wash ethanol from the effluent, ethanol-rich wash water from the effluent washer being returned to said column for codistillation with said crude aqueous ethanol.

4. A process according to claim 3 wherein said liquid product stream contains ethyl t-alkyl ether and C₅+ hydrocarbon.

5. A process according to any preceding claim wherein the etherification catalyst is an ion exchange resin.

6. A process according to any preceding claim wherein the crude aqueous ethanol contains up to 90 wt% water and the extractant comprises a major proportion of C₄-C₅ isoalkenes.

7. A process according to any preceding claim wherein said C₄+ isoalkene is derived from FCC light naphtha.

8. A process according to any preceding claim wherein said crude aqueous ethanol is derived from biomass.

## Patentansprüche

1. Verfahren zur Herstellung von Ethyl-tert.-alkylethern aus rohem wäßrigem Ethanol, welches umfaßt: Leiten des rohen Methanols zu einer Destillationskolonne, wodurch ein Kopfproduktstrom bereitgestellt wird, der die Hauptmenge von Ethanol und eine geringe Wassermenge enthält, Kontakt des Kopfproduktstroms mit einem flüssigen Kohlenwasserstoff-Extraktionsmittel, das reich an C₄⁺-Isoalken ist, bei Flüssigkeitsextraktionsbedingungen, wodurch eine wäßrige Phase, die eine geringe Ethanolmenge und die Hauptmenge von Wasser enthält, und eine organische Phase bereitgestellt werden, die das Extraktionsmittel und das restliche Ethanol enthält und im wesentlichen wasserfrei ist, Rückführung der wäßrigen Phase als Rückfluß zur Destillationskolonne und Kontakt von Ethanol und C₄⁺-Isoalken aus der organischen Phase mit einem sauren Veretherungskatalysator bei im wesentlichen wasserfreien Reaktionsbedingungen, wodurch ein Abfluß erzeugt wird, der Ethyl-tert.-alkylether enthält.

2. Verfahren nach Anspruch 1, wobei auf der Basis von Ethanol der Kopfproduktstrom mindestens 5 Gew.-% Wasser und die organische Phase nicht mehr als etwa 1,5 Gew.-% Wasser enthalten.

3. Verfahren nach einem der vorstehenden Ansprüche, wobei der Abfluß aus der Veretherungsreaktion destilliert wird, wodurch der Ethyl-t-alkylether im flüssigen Produktstrom gewonnen wird, wobei der unreagierte leichte C₄-Kohlenwasserstoff und Ethanol in einem Kopfproduktstrom gewonnen werden, der in einem Wäscher für den Abfluß mit den flüssigen Wasserrückständen aus der Destillationskolonne in Kontakt gebracht wird, wodurch das Ethanol aus dem Abfluß gewaschen wird, wobei das ethanolreiche Waschwasser aus dem Wäscher für den Abfluß für die gleichzeitige Destillation mit dem rohen wäßrigen Ethanol zur Kolonne zurückgeführt wird.

4. Verfahren nach Anspruch 3, wobei der flüssige Produktstrom Ethyl-t-alkylether und C₅⁺-Kohlenwasserstoff enthält.

5. Verfahren nach einem der vorstehenden Ansprüche, wobei der Veretherungskatalysator ein Ionenaustauschharz ist.

6. Verfahren nach einem der vorstehenden Ansprüche, wobei das rohe wäßrige Ethanol bis zu 90 Gew.-% Wasser und das Extraktionsmittel den Hauptteil der C₄-C₅-Isoalkene umfassen.

7. Verfahren nach einem der vorstehenden Ansprüche, wobei das C₄⁺-Isoalken von FCC-Leichtbenzin stammt.

8. Verfahren nach einem der vorstehenden Ansprüche, wobei das rohe wäßrige Ethanol von einer Biomasse stammt.

## Revendications

1. Procédé de fabrication d'éthyl-tert.-alkyl éthers à partir d'une solution aqueuse d'éthanol brut comprend:
- le passage de l'éthanol brut dans une colonne de distillation pour obtenir un courant de tête renfermant une proportion majeure d'éthanol et une proportion mineure d'eau,
- la mise en contact dudit courant de tête avec un agent d'extraction hydrocarboné liquide riche en isoalkène en C₄₊, dans des conditions d'extraction liquide, pour obtenir une phase aqueuse contenant une proportion mineure d'éthanol et une proportion majeure d'eau ainsi qu'une phase organique renfermant l'agent d'extraction et le reste de l'éthanol et pratiquement exempte d'eau,
- le recyclage de la phase aqueuse en tant que reflux dans la colonne de distillation
- et la mise en contact de l'éthanol et de l'isoalkène en C₄₊ de ladite phase organique avec un catalyseur d'éthérification acide dans des conditions de réaction pratiquement anhydres pour obtenir un effluent renfermant l'éthyl-t-alkyl éther.

2. Procédé selon la revendication 1, dans lequel ledit courant de tête renferme au moins 5% en poids d'eau et ladite phase organique ne contient pas plus de 1,5% en poids d'eau, exprimés par rapport à l'éthanol.

3. Procédé selon l'une quelconque des revendications précédentes, dans lequel l'effluent provenant de l'étape d'éthérification est distillé pour récupérer l'éthyl-t-alkyl éther dans un courant de produit liquide, les hydrocarbures légers en C₄ n'ayant pas réagi et l'éthanol étant récupérés dans un courant en tête qui est mis au contact dans une unité de lavage avec l'eau des produits de cuve de ladite colonne de distillation pour retirer par lavage l'éthanol de l'effluent, les eaux de lavage enrichies en l'éthanol provenant du lavage étant envoyées dans ladite colonne pour être codistillées avec ladite solution aqueuse d'éthanol brut.

4. Procédé selon la revendication 3, dans lequel ledit courant de produit liquide renferme de l'éthyl-t-alkyl éther et des hydrocarbures en C₅₊.

5. Procédé selon une quelconque des revendications 1 à 4, dans lequel le catalyseur d'éthérification est une résine échangeuse d'ions.

6. Procédé selon une quelconque des revendications 1 à 5, dans lequel l'éthanol brut aqueux renferme jusqu'à 90% d'eau et dans lequel l'agent d'extraction renferme une proportion majeure d'isoalkylènes en C₄ à C₅.

7. Procédé selon une quelconque des revendications 1 à 6, dans lequel lesdits isoalkylènes en C₄₊ proviennent d'un naphta léger FCC.

8. Procédé selon une quelconque des revendications 1 à 7, dans lequel ladite solution aqueuse d'éthanol brut est obtenue depuis une biomasse.
